(19) Europäisches Patentamt

European Patent Office

Office européen des brevets

(11) **EP 1 416 403 A2**

(12) **EUROPEAN PATENT APPLICATION**

(43) Date of publication:
06.05.2004 Bulletin 2004/19

(51) Int Cl.⁷: **G06F 17/50**

(21) Application number: **03256822.2**

(22) Date of filing: **28.10.2003**

| | |
|---|---|
| (84) Designated Contracting States:<br>**AT BE BG CH CY CZ DE DK EE ES FI FR GB GR<br>HU IE IT LI LU MC NL PT RO SE SI SK TR**<br>Designated Extension States:<br>**AL LT LV MK** | • **Mishra, Sanjay**<br>**Evansville, Indiana 47712 (US)**<br>• **Amladi, Vinod**<br>**Bangalore 560 017 (IN)**<br>• **Reddy, Dagumati Dayakara c/o G.S. Raghava<br>Raju**<br>**Bangalore 560 032 (IN)**<br>• **Saini, Pooja**<br>**Uttaranchal 248 001 (IN)** |
| (30) Priority: **28.10.2002 US 281658** | |
| (71) Applicant: **GENERAL ELECTRIC COMPANY**<br>**Schenectady, NY 12345 (US)** | (74) Representative: **Szary, Anne Catherine, Dr. et al<br>London Patent Operation<br>General Electric International, Inc.<br>15 John Adam Street<br>London WC2N 6LU (GB)** |
| (72) Inventors:<br>• **Doganaksoy, Necip**<br>**Clifton Park, New York 12065 (US)**<br>• **Gardner, Martha M.**<br>**Niskayuna, New York 12304 (US)** | |

(54) **Systems and methods for designing a new material that best matches a desired set of properties**

(57) Material creation systems and methods for quickly identifying which existing experimental run, or newly-created material, best matches a desired set of properties are described so that product development time can be minimized. Users may input the properties they desire in a material, the acceptable values of those properties, the goals for each property, and a priority value for each property. Preliminary matching existing experimental runs may be retrieved from an experimental run database. One of four desirability functions may then be utilized to calculate a scored property value for each property of each existing experimental run. The scored property value may then be weighted to account for the priority value assigned to each property. The results may then be sorted in descending order based on their overall match scores, and output to the user so the best matching existing experimental run(s) is readily identifiable by the user. Additionally, new materials may be predicted, scored, weighted and sorted so that a better match of the desired properties may be created.

**EP 1 416 403 A2**

**Description**

The present invention relates generally to systems and methods for designing a new material that matches a set of properties. More specifically, the present invention relates to material creation systems and methods that quickly create/design a new material that best matches a desired set of properties so that product development time can be shortened.

Often times, when new products are being designed, a material having certain properties is desired, but it may not be readily apparent which combination of ingredients will produce a material that best meets those properties. Therefore, experimentation may be required to find the best combination of ingredients and/or processing conditions that will yield such a material. Such experimentation may be a time consuming and expensive endeavor, thereby making experimentation undue and unfeasible in many instances.

Current systems and methods for designing materials that possess desired properties mainly utilize statistically designed sets of experiments - called design spaces. Design spaces typically represent a designed experiment around a common set of ingredients, and may include the following as independent (or manipulated) variables: relative proportions of the ingredients, quality parameters of these ingredients, and processing parameters, with the final material properties serving as the dependent (or response) variables. Each experimental run in a design space represents a combination of the independent variables with measured dependent variables as output. A design space will have multiple experimental runs, some of which are unique and some of which are replicated in order to be able to assess the inherent error in experiments. Many current design spaces reside as single files on the computer of the original product developer, and thus, are not searchable or of use to other product developers. Even if a product developer has access to multiple design spaces having properties that are somewhere in the vicinity of the desired properties of a new material, someone has to sort through these design spaces and decide which experimental run(s) most closely matches the desired properties. Often times, this may be done in an engineer's head, or the decision may be based on instinct, knowledge and experience or other unscientific means. As such, further testing or experimentation is often required to find a new material that best matches the desired properties overall. This is further complicated by the fact that an experimental run within a design space may very closely match one or more desired property values, while not very closely matching other desired property values at all. Therefore, in the absence of appropriate tools and systems it is often difficult to tell which experimental run(s) in the list will best match the desired properties overall.

Sometimes, existing experimental runs may not be the best match of the desired properties; it may be necessary to create/design a new material to provide the best match. However, existing systems and methods do not easily allow new materials to be created/designed without additional experimentation so that a better match of the desired properties can be obtained.

As such, there is presently no quick and easy way to create a new material that best matches the desired properties overall. Thus, there is a need for systems and methods that allow one to quickly identify which existing experimental run(s), or which newly-created material(s), most closely matches the desired properties overall, thereby allowing product development cycle times to be significantly reduced. There is also a need for such systems and methods to allow users to search only certain design spaces (i.e., only those design spaces containing a certain raw material such as polycarbonates). There is also a need for such systems and methods to be automated using a computer. There is yet a further need for such systems and methods to be accessible to users via an Intranet. There is also a need for such systems and methods to take all the desired properties into account collectively when calculating which experimental run(s) best matches the desired properties overall. There is still a further need for such systems and methods to utilize desirability functions to score existing experimental runs according to how well they match each individual desired property value. There is also a need for such systems and methods to only provide an overall match score for an experimental run if none of the experimental run's individual scores are zero. There is also a need for such systems and methods to allow properties having higher priorities to be given greater weight than properties having lower priorities when the overall match score of the experimental run is being calculated. There is yet a further need for such systems and methods to utilize models (also called transfer functions) to predict new materials that may better match the desired properties than existing experimental runs do. Furthermore, there is a need for such systems and methods to allow materials to be ranked in descending order according to their calculated overall match score, so that the material(s) that best matches the desired properties, and whether it is an existing experimental run or a new material, is readily identifiable by a user. Finally, there is a need for such systems and methods to allow new design spaces to be carefully and deliberately planned, designed and created so that invalid data is kept out of the system.

Accordingly, the above-identified shortcomings of existing systems and methods are overcome by embodiments of the present invention. This invention relates to material creation systems and methods that allow one to quickly identify which existing experimental run(s), or which newly-created material(s), most closely matches a desired set of properties overall, thereby allowing new product development cycle times to be significantly reduced. An embodiment of this invention comprises systems and methods that utilize a computer to automatically search a database of experimental runs (wherein the database may contain several different design spaces) and calculate which existing experimental run(s) therein best matches the desired set of properties overall. As used herein, "design space" means a collection of past experimental data that is grouped together based on common independent variables in a structured format. Each design space consists of several experimental runs (i.e., each run is one specific formulation that has been tested). In embodiments, a computer may also be used to create a new (i.e., theoretical) material or formulation if a better match of the desired properties can be obtained from a new material than from an existing experimental run. Models (i.e., transfer functions) may be utilized to predict such new materials/formulations. Embodiments also allow the user to elect to search only selected design spaces (i.e., only those design spaces containing a certain raw material such as polycarbonates). In some embodiments, the systems and methods of this invention may be accessible to users via a personal computer, and/or via an intranet. Embodiments of the systems and methods of this invention may take all the desired properties into account collectively when calculating which experimental run(s) or new material(s) best matches the desired set of properties overall. In embodiments of this invention, the systems and methods may utilize desirabilty functions (Derringer and Suich, "Simultaneous Optimization of Several Response Variables," *Journal of Quality Technology*, Vol. 12, pp. 214-219, 1980) to score existing experimental runs and/or new materials according to how well they match each individual desired property value. Some embodiments only provide an overall match score for an experimental run if none of the experimental run's individual property scores are zero. Furthermore, properties having higher priorities can be given greater weight than properties having lower priorities when the overall match score of the experimental run or new material is being calculated. Embodiments of the systems and methods of this invention can allow materials to be ranked in descending order according to their calculated overall match score, so that the material(s) that best matches the desired properties is readily identifiable by a user. Embodiments also identify whether the best matching material(s) are existing experimental runs or new (i.e., theoretical) materials. Finally, embodiments of the systems and methods of this invention may allow new design spaces to be carefully and deliberately planned, designed and created so that invalid data is kept out of the system.

When designing a new product, often times a material possessing certain properties may be desired. In anticipation of this, carefully planned design spaces have been created so that existing experimental materials/formulations may be searched, and new materials/formulations can be interpolated and created from the existing experimental materials/formulations, so that a material/formulation that best matches a desired set of properties can be identified. As used herein, "material" and "formulation" are used interchangeably to mean the same thing. Embodiments of the systems and methods of this invention allow a user to select raw material classifications they desire to search. For example, users may only wish to search design spaces containing certain raw materials, such as, for example, polycarbonates.

Users may also input or select the various properties and property values that they desire in a material. For example, users may be able to select the properties they desire in a material. Users may also be able to select what they wish to do with the property values, such as maximize the property value, minimize the property value, hit a target point value for the property value, or keep property values within a given range of acceptable values. In embodiments, users may also be able to select a priority for each desired property, such as high, medium or low.

Once the design spaces to be searched, and/or the properties and the desired property variables, and/or the priorities for each property are selected, a search for the closest matching experimental run or new material can begin. In one embodiment, the first step involves searching a database of experimental runs to find out which design spaces contain user-selected raw materials. The user may then be given the opportunity to select which of these design spaces to retain for advanced searching and scoring. Other embodiments may begin by having the user select which properties they desire in a material.

Next, the user may input the actual property values that are desired, the property units, what the goal is for each property (i.e., maximize the property value, minimize the property value, hit a target point value for the property value, or keep property values within a given range of acceptable values), and what the priority/importance is for each property (i.e., high, medium, low). Thereafter, the database of experimental runs (i.e., the design spaces) may be searched to find out which design spaces contain experimental runs that possess the desired properties. In embodiments, if one property is not met by an experimental run in a design space, that experimental run will not be scored. Furthermore, if none of the experimental runs in a design space meets one or more of the desired property requirements, then the entire design space may be blocked (i.e., nothing in the design space will be scored). Users may then select which design spaces they wish to have scored. A score may then be calculated for each property value, and an overall match score may be calculated for each experimental run, indicating how well the experimental run matches the desired properties. The highest overall match score in each design space, and the property having the lowest individual property score in each design space may then be displayed to users. Users may then select which design spaces they wish to have scored using transfer functions, so that predicted formulations (i.e., new materials that may better match the desired properties than do existing experimental runs) can be created. This invention will then perform the transfer function scoring and output the results to the user. This output may consist of overall match scores for actual experimental runs that exist within the design spaces, as well as newly-created or predicted materials, that theoretically match the desired properties. In embodiments, users select one design space at a time to score via the transfer functions, then they may be given the opportunity to compare the results of various scored design spaces. In all of the output to the user, the materials (whether they are experimental runs or new materials) may be sorted in descending order based on their overall match scores so that a user can easily identify which material matches all the desired properties the best.

In one embodiment, desirability functions are used to calculate a score for each property value. These desirability functions determine the degree of similarity between the desired property values and the actual property values of existing experimental runs. There are four different desirability functions utilized by the present invention, depending on what goal is selected by the user for each desired property. For example, if the user desires to maximize a property value, one desirability function is utilized to calculate a score for that property value. If the user desires to minimize a property value, a second desirability function is utilized to calculate the score for that property value. If the user desires to hit a target point value for the property value, a third desirability function is utilized to calculate the score for that property value. Finally, if the user desires to keep property values within a given range of acceptable values, a fourth desirability function is utilized to calculate the score for that property value.

In embodiments, each score may also be weighted to account for the priority selected for that property. For example, if a high priority is selected for a property, that property may be assigned a higher value than one having a lower priority so that when the overall match score is calculated, these priorities are taken into account.

In embodiments, the overall match score may take all the property values into account collectively. The calculations described above may be performed automatically by a computer, or they may be performed manually. Furthermore, the systems and methods may be designed so that, once a user selects the desired properties and acceptable property values, a database of design spaces is automatically searched and the best matching experimental run(s) therein is located, or a newly-created better matching material is automatically designed.

The present invention has all the advantages of existing material creation systems and methods, but it requires less experimentation and laboratory time, thereby reducing product development cycle times so that new products can get to market quicker.

One embodiment of the present invention comprises a method for selecting an existing experimental run or creating a new material that most closely matches a desired set of properties. This method may comprise obtaining at least one input parameter from a user; retrieving actual property values for at least one preliminary matching existing experimental run from a global data repository; determining how well each preliminary matching existing experimental run matches a desired set of property values; and outputting the results to the user. This determining step may further comprise scoring each property value of each preliminary matching existing experimental run to create a scored property value; and calculating an overall match score for each preliminary matching existing experimental run. Calculating an overall match score may comprise weighting each scored property value by taking a weight value for each property into account to create a weighted scored property value; multiplying each weighed scored property value together; and raising the multiplied quantity to 1/(sum of all the priorities). The method may also comprise sorting the preliminary matching existing experimental runs by their respective overall match scores prior to outputting the results to the user. Additionally, the method may comprise predicting at least one new material that may more closely match the desired set of properties than any existing experimental run. The new materials may also be scored and sorted along with the preliminary matching existing experimental runs so that the combined results thereof can be output to the user. A new product or material that best matches the user-specified properties and property values may then be created based on these results.

Another embodiment of the present invention comprises a system for selecting an existing experimental run or creating a new material that most closely matches a desired set of properties. This system may comprise a means for obtaining at least one input parameter from a user; a means for retrieving actual property values for at least one preliminary matching existing experimental run from a global data repository; a material selection algorithm operable for determining how well each preliminary matching existing experimental run matches a desired set of property values; and a means for outputting the results to the user. This material selection algorithm may be further operable for scoring each property value of each preliminary matching existing experimental run to create a scored property value; and calculating an overall match score for each preliminary matching existing experimental run. The material selection algorithm may also be operable for sorting the preliminary matching existing experimental runs by their respective overall match scores prior to outputting the results to the user. Additionally, the system may comprise a material prediction algorithm operable for predicting at least one new material that may more closely match the desired set of properties than any existing experimental run. The system may also comprise means for scoring and sorting the new materials along with the preliminary matching existing experimental runs so that the combined results thereof can be output to the user. A new product or material that best matches the user-specified properties and property values may then be created based on these results.

Further features, aspects and advantages of the present invention will be more readily apparent to those skilled in the art during the course of the following description, wherein references are made to the accompanying figures which illustrate some preferred forms of the present invention, and wherein like characters of reference designate like parts throughout the drawings.

Embodiments of the systems and methods of the present invention are described herein below, by way of example, with reference to various drawings and graphical representations thereof, in which:

Figure 1 is a flowchart showing the material properties retrieval and overall match score calculations that are performed in one embodiment of this invention;

Figure 2 is a graph showing the desirability function applied when a user desires to maximize a property value, assuming a linear approach to the goal;

Figure 3 is a graph showing the desirability function applied when a user desires to minimize a property value, assuming a linear approach to the goal;

Figure 4 is a graph showing the desirability membership function applied when a user desires to hit a target point value for the property value, assuming a linear approach to the goal;

Figure 5 is a graph showing the desirability function applied when a user desires to keep property values within a given range of acceptable values; and

Figure 6 is a schematic diagram showing the three-tiered architecture of one embodiment of a system for selecting an existing experimental run or creating a new material that best matches a desired set of properties.

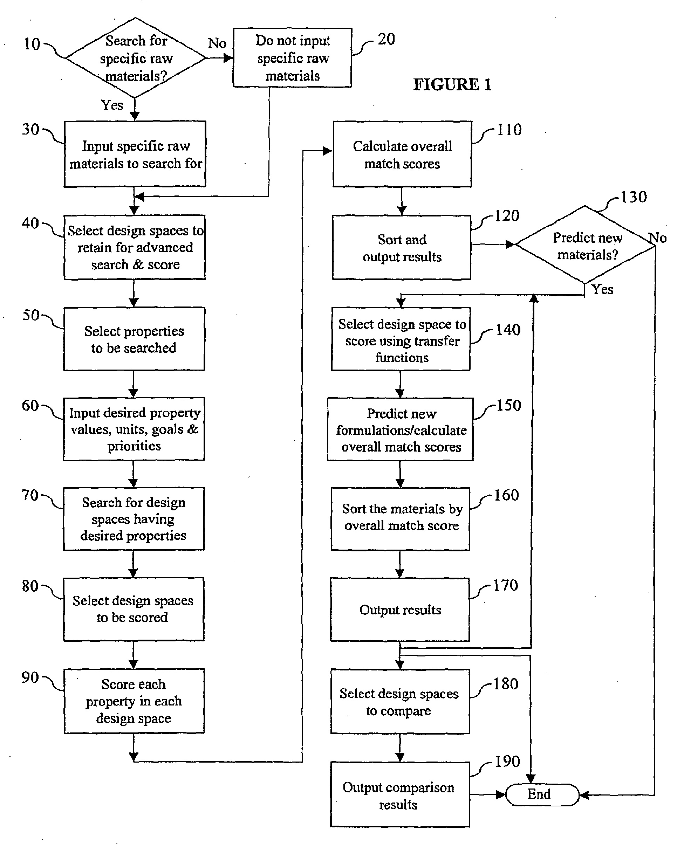

For the purposes of promoting an understanding of the invention, reference will now be made to some preferred embodiments of the present invention as illustrated in FIGURES 1-6, and specific language used to describe the same. The terminology used herein is for the purpose of description, not limitation. Specific structural and functional details disclosed herein are not to be interpreted as limiting, but merely as a basis for the claims as a representative basis for teaching one skilled in the art to variously employ the present invention. Any modifications or variations in the depicted material creation systems and methods, and such further applications of the principles of the invention as illustrated herein, as would normally occur to one skilled in the art, are considered to be within the spirit of this invention.

The present invention comprises material creation systems and methods that allow one to quickly identify which existing experimental runs, or which newly-created materials, most closely match a desired set of properties overall so that new product development time can be reduced. In one embodiment, the material creation method comprises the steps shown in Figure 1. First a user may decide if they wish to search for specific raw materials 10, such as, for example, polycarbonates, and they can input the specific raw materials they wish to search for 30. If the user does not wish to search for a specific raw material, they do not need to input specific raw materials 20. The present invention may then display a list of the design spaces that contain those user-specified raw materials. Next, users may select which design spaces they wish to retain for advanced searching and scoring 40. Then, users can input the desired properties 50. Users may also input the desired property values, property units, what the goals are for each property (i.e., maximize the property value, minimize the property value, hit a target point value for the property value, or keep property values within a given range of acceptable values), and what the priority is for each property (i.e., high, medium, low) 60. These properties may include mechanical properties, thermal properties, impact properties, or other desired properties or materials. Such properties may include one or more of the following non-limiting properties: flexural modulus, flexural strength, tensile elongation (strain), tensile modulus, tensile strength, crystallization temperature, HDT at 264 psi (1.8 MPa), HDT at 66 psi (0.45 MPa), melt flow rate at 300°C/1.2kg, melt viscosity at 266°C/10 kg, melt viscosity at 266°C/5 kg, melt volume rate at 250°C/5 kg, melt volume rate at 265°C/10 kg, melt volume rate at 265°C/2.16 kg, melt volume rate at 265°C/5 kg, Vicat B/120, Notched Izod impact - notched at 23°C, Notched Izod impact - unnotched at 23°C, Dynatup impact total energy at 23 °C, Izod impact - notched at -20°C, Izod impact - notched at -25°C, Izod impact - notched at -30°C, Izod impact - notched at -40°C, Izod impact - notched at 0°C, Izod impact - notched at 23°C, Izod impact - unnotched at 23°C, dielectric strength, flame out time (average of 10 burns) at 1.6mm, gloss, haze, mold shrinkage - flow, mold shrinkage - xflow, pFTP at 1.6mm, specific gravity, yellowness index, filler/fibers, impact modifiers, PBT, flame retardants, other polyesters, PC, stabilizers - light, and stabilizers - others. Once the user decides which properties they desire in a material, then the user inputs the desired or acceptable values for that property, and also selects which units are desired for each property (i.e., SI units or US/British units). For example, the user may desire to maximize the property value, minimize the property value, hit a target point value for the property value, or keep property values within a given range of acceptable values. The user may also input the priority assigned to each property. The priorities may comprise high, medium and low.

Now, the search for the best matching experimental run(s) or new material can begin. In this embodiment, the present invention then searches a global data repository or database of design spaces for design spaces having the desired properties 70, and displays these results to the user. This global data repository may comprise data for experimental runs from all around the globe, instead of just comprising data from one region of the globe. For example, embodiments of this invention comprise data for experimental runs available in North America, Europe, Asia, etc., all combined into one searchable global data repository. Preferably, the design spaces are searchable only by approved product designers because the information contained in the design spaces may be confidential and undisclosed to the general public. Users may then select which design spaces they wish to have scored 80.

In embodiments of this invention, background calculations may be performed. Users may or may not even be aware that these background calculations are occurring. For example, if property values for an experimental run are retrieved from the global data repository in SI units, but the user wants the units to be displayed in British or U.S. units, embodiments of the invention may convert the retrieved units to the appropriate desired units before displaying them to the user. Also, data may be normalized as needed so that testing methods used to measure a given property in one location can be normalized to testing methods used to measure that same property in another location. Other background calculations may also be performed. For example, if results of a specific testing method are requested by a user, but that test has not been performed and entered into the database, then if a similar test has been conducted, the desired results may in some cases be calculated from the actual results of the similar test.

In this embodiment, this invention will not score an experimental run if the experimental run lacks data for even one property.

Once the user selects which design spaces they wish to have scored, a score for each property value of each experimental run can be calculated 90. In an embodiment, the score is calculated via one of four possible desirability functions. If a user wishes to maximize a property value (i.e., if the goal is to maximize the property value), the score for that property value may be calculated using a first desirability function:

| If the actual property value (APV) is: | Use this Desirability Function: |
|---|---|
| APV < MIN | Score = 0 |
| APV > MAX | Score = 1 |
| Otherwise | $\text{Score} = \left[ \dfrac{APV - MIN}{MAX - MIN} \right]^{x}$ |

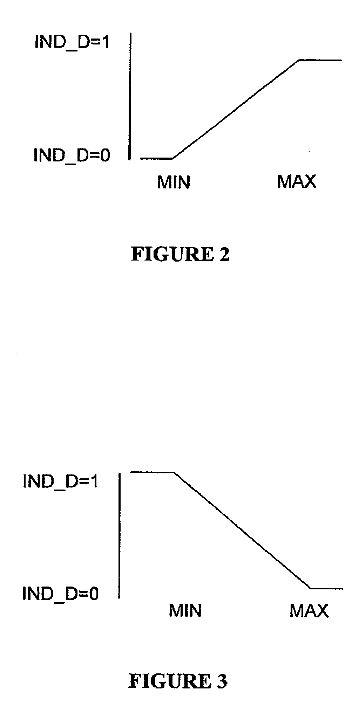

where APV = actual property value, MAX = user-specified maximum acceptable property value, MIN = user-specified minimum acceptable property value, and x = the weight value. The weight value (x) is used to change the shape of the desirability function. Weight values typically range from about 0.1 to about 10. A weight value of 1 yields a linear score between MIN and MAX, and is the typical weight value used. Weight values less than 1 de-emphasize the goal, whereas weight values greater than 1 strongly emphasize the goal. A graphical representation of this desirability function, with a weight value of 1, is shown in Figure 2.

If a user wishes to minimize a property value (i.e., if the goal is to minimize the property value), the score for that property value may be calculated using a second desirability function:

| If the actual property value (APV) is: | Use this Desirability Function: |
|---|---|
| APV > MAX | Score = 0 |
| APV < MIN | Score = 1 |
| Otherwise | $\text{Score} = \left[ \dfrac{MAX - APV}{MAX - MIN} \right]^{x}$ |

where APV = actual property value, MAX = user-specified maximum acceptable property value, MIN = user-specified minimum acceptable property value, and x = the weight value. A weight value of 1 is typically used. A graphical representation of this desirability function, with a weight value of 1, is shown in Figure 3.

If a user wishes to hit a target point value for a property value (i.e., if the goal is to hit a target point value for the property value), the score for that property value may be calculated using a third desirability function:

| If the actual property value (APV) is: | Use this Desirability Function: |
|---|---|
| APV ≥ DPV and APV ≤ MAX | $Score = \left[\dfrac{MAX - APV}{MAX - DPV}\right]^{X}$ |
| APV > MAX | Score = 0 |
| APV ≤ DPV and APV ≥ MIN | $Score = \left[\dfrac{APV - MIN}{DPV - MIN}\right]^{X}$ |
| APV < MIN | Score = 0 |

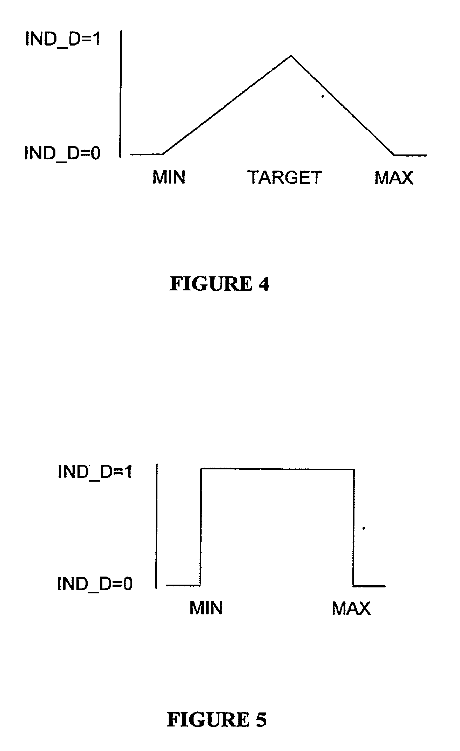

where APV = actual property value, MAX = user-specified maximum acceptable property value, MIN = user-specified minimum acceptable property value, DPV = user-specified desired property value, and x = the weight value. A weight value of 1 is typically used. A graphical representation of this desirability function, with a weight value of 1, is shown in Figure 4.

Finally, if a users wishes to keep property values within a given range of acceptable property values (i.e., if the goal is to keep property values within a given range of acceptable property values), the score for that property value may be calculated using a fourth desirability function.

| If the actual property value (APV) is: | Use this Desirability Function: |
|---|---|
| MIN ≤ APV ≤ MAX | Score = 1 |
| Otherwise | Score = 0 |

where APV = actual property value, MAX = maximum acceptable property value, and MIN = minimum acceptable property value. A graphical representation of this piecewise linear desirability function is shown in Figure 5.

Once a score for each property value is calculated, an overall score can be calculated. This overall score may take the relative priorities of each property into account. For example, in this embodiment, if a property is given a high priority, a priority value of 5 is assigned to that property; if a property is given a medium priority, a priority value of 3 is assigned to that property; and if a property is given a low priority, a priority value of 1 is assigned to that property. An overall match score can be calculated 110 by first raising each individual score to its priority to create a weighted score for each property, then multiplying these weighted scores for each property together, and then raising this multiplied quantity to 1/(sum of all the priorities) as follows:

$$OverallMatchScore = \left[\prod_{i=1}^{n}(Score_i)^{priority_i}\right]^{1/\sum_{i=1}^{n} priority}$$

The existing experimental runs may then be sorted 120 in descending order of their overall match scores. Finally, a list of the best matching experimental runs in the design space may be output to the user 120 so the experimental run that matches the best is listed at the top of the output list so it can be easily identified by the user.

Thereafter, in this embodiment, users may be given the opportunity to predict new materials 130. Users may select which design spaces, if any, they wish to have scored using transfer functions 140 so that new, possibly better matching, materials can be created/predicted 150. Overall match scores may then be calculated for these predicted materials 150, and all the materials (both existing and predicted) within the design space may be sorted by their overall match scores 160, and the results may be output to the users 170. In this embodiment, only one design space at a time can be scored using transfer functions, but embodiments may be designed so that several design spaces can be scored simultaneously using transfer functions. Once all the desired design spaces are scored with transfer functions, users may be given the opportunity to select various design spaces to compare 180. The comparison results may then be output to the users 190.

To further clarify these calculations, reference will now be made to the table below, which shows all the above-described calculations for a material. First, the properties that were desired were selected - tensile strength, Notched Izod Impact - notched at 23°C, HDT @ 264 psi (1.8 MPa), and molecular weight. Next, the units desired for each of these properties, the desired values for each of these properties, the goals for each of these properties, and the priorities for each of these properties were selected as follows:

| Properties Desired: | Goal | Priority Value |
|---|---|---|
| 40 MPa < Tensile Strength < 47 MPa | Maximize | High (5) |
| Notched Izod Impact = 13 kJ/m$^2$ <br> (Min = 11 kJ/m$^2$, Max = 15 kJ/m$^2$) | Seek Target | Low (1) |
| 80°C < HDT < 92°C | Minimize | Medium (3) |
| 24000g/gmole < Molecular Weight < 26000g/gmole | In Range | Medium (3) |

For this embodiment, the units selected were SI units. The actual value of tensile strength from the database of design spaces for this particular material was 46 MPa.

As previously discussed, the score for this property is calculated as follows (using a weight of 1):

$$\text{Score} = \left[\frac{\text{APV} - \text{MIN}}{\text{MAX} - \text{MIN}}\right]^{X} = \left[\frac{46 - 40}{47 - 40}\right]^{1} = .8571$$

The actual value of Notched Izod Impact from the database of design spaces for this particular experimental run was 13.2 kJ/m$^2$. The score for this property can be calculated as follows:

$$\text{Score} = \left[\frac{\text{MAX} - \text{APV}}{\text{MAX} - \text{DPV}}\right]^{X} = \left[\frac{15 - 13.2}{15 - 13}\right]^{1} = 0.9$$

The actual value of HDT from the database of design spaces for this particular experimental run was 90.3°C. The score for this property can be calculated as follows:

$$\text{Score} = \left[\frac{\text{MAX} - \text{APV}}{\text{MAX} - \text{MIN}}\right]^{X} = \left[\frac{92 - 90.3}{92 - 80}\right]^{1} = 0.1417$$

Finally, the actual value of Molecular Weight from the database of design spaces for this particular experimental run was 25000g/gmole. The score for this property equals 1.

Next, an overall match score for this experimental run can be calculated as follows:

$$OverallMatchScore = \left[ \prod_{i=1}^{n} \left( Score_i \right)^{priority_i} \right]^{1/\sum_{i=1}^{n} priority_i}$$

Overall Match Score = $[(.8571^5)(0.9^1)(0.1417^3)(1^3)]^{1/(5+1+3+3)} = 0.001184^{1/12} = .5703$

The overall match score rates how well a material fits the desired property values. Each material will have an overall match score ranging from 0 to 1.0, depending on how well it matches the desired properties. An overall match score of 1.0 means the material matches all the desired properties perfectly, while an overall match score of 0.0 means the material does not match the desired properties at all.

In this embodiment, once the experimental runs in each design space are scored, sorted and output to the user, the user may be given the opportunity to select which design spaces they wish to have scored using transfer functions so that predicted formulations (i.e., new materials that may better match the desired properties than do existing experimental runs) can be created. Before the transfer functions are applied, a mesh of predicted new materials may first be generated. The mesh is really a constrained optimization problem solution. One exemplary non-limiting way in which this constrained optimization problem may be solved is now described. Those skilled in the art will recognize that other solutions to this problem exist. First, if you have $n$ components, you can build a mesh around all $n$ components using a user-desired number of increments ($x$). For example, if a user has a design space with three ingredients as factors and wants a particular ingredient (i.e., A) to comprise about 10-40% of the material, and they want 3 increments, then the mesh will be built to show varying amounts of A in the material (i.e., 10% A, 20% A, 30% A and 40% A). The total number of potential new materials created by this mesh will be $(x+1)^n = 4^3$, or 64 potential new materials. In this embodiment, users may select 3-7 increments, but embodiments may be designed so that other numbers of increments could be selected. Next, the n components in each potential new material are summed and any combinations having a sum less than 100 are ignored. Any combinations having a sum equal to 100 are kept as potential new materials, so long as each component falls within its desired range. For any combinations having a sum over 100, 100 is subtracted from the total and this lesser quantity is then subtracted from each of the individual components one at a time to see if the resulting combination will fall within the desired bounds for each component.

To further clarify this mesh generation, a subset of this mesh will now be discussed. Let's say a user decides that they desire a material to have about 10-40% component A, about 20-50% component B, and about 10-70% component C. The sum total of these components must equal 100%.

| New Material # | A | B | C | Sum | Action |
|---|---|---|---|---|---|
| 1 | 10 | 20 | 30 | 60 | Ignore |
| 2 | 10 | 30 | 10 | 50 | Ignore |
| 3 | 40 | 30 | 50 | 120 | Subtract 20 |
| 3A | 20 | 30 | 50 | 100 | Keep as is |
| 3B | 40 | 10 | 50 | N/A | Ignore |
| 3C | 40 | 30 | 30 | 100 | Keep as is |
| 4 | 30 | 40 | 70 | 140 | Subtract 40 |
| 4A | -10 | 40 | 70 | N/A | Ignore |
| 4B | 30 | 0 | 70 | 100 | Ignore |
| 4C | 30 | 40 | 30 | 100 | Keep as is |
| 5 | 10 | 40 | 50 | 100 | Keep as is |

Potential new material #1 and #2 both get ignored because the sum of their components is less than 100.

New materials #3 gets modified because the sum of its components is greater than 100. Twenty is subtracted from each component, one at a time (i.e., new materials 3A, 3B and 3C), to see if the component still falls within the desired range. New material 3A is kept as a potential new material because each component falls within its desired range and the total of the components equals 100. New material 3B is ignored because the percentage for component B falls below the desired range of 20-50%. New material 3C is kept as a potential new material because each component falls within its desired range and the total of the components equals 100.

New materials also #4 gets modified because the sum of its components is greater than 100. Forty is subtracted from each component, one at a time (i.e., new materials 4A, 4B and 4C), to see if the component still falls within the desired range. New material 4A is ignored because the percentage for component A falls below the desired range of 10-40%. New material 4B is ignored because the percentage for component B falls below the desired range of 20-50%. New material 4C is kept as a potential new material because each component falls within its desired range and the total of the components equals 100.

New material 5C is kept as a potential new material because each component falls within its desired range and the total of the components equals 100.

Next, all combinations are checked to ensure that no duplications exist. Once this mesh of potential new materials is generated, transfer functions may be applied to predict the property values. The transfer functions applied in this invention generally comprise, although are not limited to, polynomial models relating the properties to the independent variables such as the relative proportions of ingredients, processing parameters, and raw material quality parameters. In cases where there is a sum total constraint on the percentage or proportion of ingredients, a special polynomial form called a Scheffe polynomial model is generally employed (Cornell, J., EXPERIMENTS WITH MIXTURES, publ. by John Wiley & Sons, NY, 1990). Transfer functions can also be physical, rather than empirical, models. Additionally, transfer functions can be developed not just for the mean value of the property, but also for the standard deviation of the property using techniques such as propagation of error and/or direct calculation of standard deviations via an inner-outer array approach (Myers, R. H. and Montgomery, D. C., RESPONSE SURFACE METHODOLOGY, publ. by John Wiley & Sons, NY, 1995). These transfer functions interpolate the data in the spaces between the existing experimental runs to design/create new materials that may better match the desired properties overall than existing experimental runs do. In this particular embodiment, only one design space at a time can be scored using transfer functions, but other embodiments may be designed so that multiple design spaces could all be scored with transfer functions simultaneously. Once these new formulations/materials are predicted, overall match scores may be calculated for each of these materials as well, just as described for existing experimental runs above. Next, the materials may be sorted by their respective overall match scores, and the results may be output to the user. These results preferably comprise data of existing experimental runs as well as predicted materials, sorted accordingly, so users can easily identify whether an existing experimental run or a newly-predicted material best matches the desired set of properties. If desired, a user may then select design spaces to compare. For example, since only one design space at a time can be scored with transfer functions in this embodiment, the user can have each design space scored with transfer functions, and then compare the results of all the scored design spaces afterwards.

Embodiments of this invention also comprise methods that allow new design spaces to be carefully and deliberately planned, designed and created so that invalid data is kept out of the system. These methods may comprise suggesting guidelines on structuring experiments, recommended testing, creating models, and interpreting results for new design spaces.

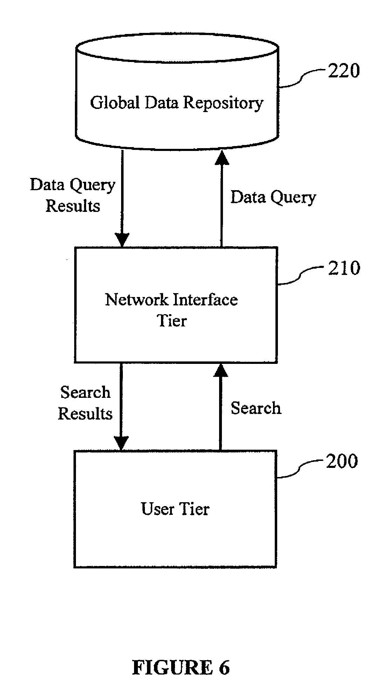

Embodiments of the present invention also comprise material creation systems. In one embodiment, the material creation system comprises a three-tier architecture as shown in Figure 6. The three tiers in this embodiment include the user tier 200, the network tier 210 and the database tier 220. The user tier in this embodiment allows a user to input or select various input parameters. Some non-limiting examples of these various input parameters comprise: any specific raw materials the user may wish to search for, one or more design spaces containing these specific raw materials that the user wishes to retain for advanced searching, one or more design spaces the user wishes to retain for scoring, one or more properties the user wishes to be searched, what the acceptable property values are for each property being searched, what the goal is for each property being searched, and/or a priority value for each property being searched. This user tier may contain a material search interface layer implemented in any suitable manner, such as by JavaServer Pages™ (JSP) technology and/or JavaScript. In this embodiment, the network tier layer hosts the actual application that performs the material search (i.e., the network tier acts as the material search engine). The network tier accepts the user's inputs, and then performs the search/data query over the database layer. The search results may then be returned to the user via the user tier. This functionality may be achieved in any suitable manner, such as by using a web server, Java Servlet and/or Java Data Base Connectivity (JDBC™) technology.

Embodiments of this invention search a global data repository comprising any type of materials such as, for example, plastics, glasses, ceramics, and/or metals, etc. Other embodiments search a global data repository comprising various design spaces made up of engineering thermoplastics. The experimental runs contained in these design spaces are generally experimental materials, but could also contain commercially available materials. These thermoplastics may comprise, for example, polyesters, such as polyethylene terephthalate (PET), polybutylene terephthalate (PBT), polyethylene naphthalate (PEN), liquid crystal polyester (LCP) and the like, polyolefins, such as polyethylene (PE), polypropylene (PP), polybutylene or the like, styrene-type resins, etc. or polyoxymethylene (POM), polyamide (PA), polycarbonate (PC), polymethylene methacrylate (PMMA), polyvinyl chloride (PVC), polyphenylene sulfide (PPS), polyphenylene ether (PPE), polyimide (PI), polyamide imide (PAI), polyetherimide (PEI), polysulfone (PSU), polyether sulphone (PES), polyketone (PK), polyether ketone (PEK), polyether ether ketone (PEEK), polyalylate (PAR), polyethernitrile (PEN), phenol resins (novolac type or the like), phenoxy resins, fluorocarbon resins, or, furthermore, thermoplastic elastomers of a polystyrene type, a polyolefin type, a polyurethane type, a polyester type, a polyamide type, a polybutadiene type, polyisoprene type, a fluorine type or the like, or copolymers or modifications of any of the these substances, or blended resins of two or more of these substances or the like. More preferably, these thermoplastics comprise styrene-type resins, polycarbonate resins, polyphenylene ether resins, polyamide resins, polyester resins, polyphenylene sulfide resins, liquid-crystalline resins and phenol-type resins. The thermoplastics in this invention may further comprise one or more reinforcing agents such as glass, talc, mica, clay, or combinations thereof; flame retarding compounds used alone or in conjunction with a synergist; drip retarding agent(s); and/or a wide variety of other additives such as stabilizers, pigments, colorants, processing aids, antioxidants and the like.

As described above, the systems and methods of the present invention allow a user to quickly and easily identify an existing experimental run, or create/predict a new material, that closely matches desired performance criteria. Advantageously, these systems and methods may significantly speed up new product development times, allowing new products to get to market quicker than in the past.

Various embodiments of the invention have been described in fulfillment of the various needs that the invention meets. It should be recognized that these embodiments are merely illustrative of the principles of various embodiments of the present invention. Numerous modifications and adaptations thereof will be apparent to those skilled in the art without departing from the scope of the present invention. Thus, it is intended that the present invention cover all suitable modifications and variations as come within the scope of the appended claims and their equivalents.

For completeness, various aspects of the invention are now set out in the following numbered clauses:

1. A method for designing a material that most closely matches a desired set of properties, the method comprising:

obtaining at least one input parameter from a user;
retrieving actual property values for at least one preliminary matching existing experimental run from a global data repository;
determining how well each preliminary matching existing experimental run matches a desired set of property values; and
outputting the results to the user.

2. The method of clause 1, wherein the determining step comprises:

scoring each property value of each preliminary matching existing experimental run to create a scored property value; and
calculating an overall match score for each preliminary matching existing experimental run.

3. The method of clause 2, wherein calculating an overall match score comprises:

weighting each scored property value by taking a weight value for each property into account to create a weighted scored property value;
multiplying each weighted scored property value together; and
raising the multiplied quantity to 1/(sum of all the priorities).

4. The method of clause 2, further comprising:

sorting the preliminary matching existing experimental runs by their respective overall match scores prior to outputting the results to the user.

5. The method of clause 4, wherein the preliminary matching existing experimental runs are sorted in descending order based on their respective overall match scores.

6. The method of clause 1, further comprising the step of:

predicting at least one new material that may more closely match the desired set of properties than any existing experimental run.

7. The method of clause 6, further comprising the steps of:

scoring each property value of each new material to create a scored property value; and calculating an overall match score for each new material.

8. The method of clause 7, wherein calculating an overall match score comprises:

weighting each scored property value by taking a weight value for each property into account to create a weighted scored property value;
multiplying each weighted scored property value together; and
raising the multiplied quantity to 1/(sum of all the priorities).

9. The method of clause 7, further comprising the step of:

sorting the preliminary matching existing experimental runs and the new materials by their respective overall match scores prior to outputting the combined results thereof to the user.

10. The method of clause 6, wherein the predicting step comprises applying a transfer function to predict the new material that may more closely match the desired set of properties than any existing experimental run.

11. The method of clause 1, wherein the at least one input parameter comprises at least one of: a specific raw material to search for, a design space to retain for advanced searching, a design space to retain for scoring, a property to be searched, units for each property to be searched, acceptable property values for each property to be searched, a goal for each property to be searched, and a priority value for each property to be searched.

12. The method of clause 11, wherein the goal for each property to be searched comprises at least one of: maximize the property value, minimize the property value, hit a target point value for the property value, and keep the property value within a given range of acceptable property values.

13. The method of clause 11, wherein the priority value for each property to be searched comprises at least one of: high, medium and low.

14. The method of clause 2, wherein if at least one input parameter obtained from the user is maximize the property value, and if the actual property value is less than a minimum acceptable property value, then the scored property value is zero (0).

15. The method of clause 2, wherein if at least one input parameter obtained from the user is maximize the property value, and if the actual property value is greater than a maximum acceptable property value, then the scored property value is one (1).

16. The method of clause 2, wherein if at least one input parameter obtained from the user is maximize the property value, and if the actual property value is not less than a minimum acceptable property value, and if the actual property value is not greater than a maximum acceptable property value, then the scored property value may be calculated using the desirability function:

$$\text{Score} = \left[ \frac{\text{APV} - \text{MIN}}{\text{MAX} - \text{MIN}} \right]^{x}$$

wherein APV = actual property value, MAX = user-specified maximum acceptable property value, MIN = user-specified minimum acceptable property value, and x = the weight value.

17. The method of clause 2, wherein if at least one input parameter obtained from the user is minimize the property value, and if the actual property value is greater than a maximum acceptable property value, then the scored property value is zero (0).

18. The method of clause 2, wherein if at least one input parameter obtained from the user is minimize the property value, and if the actual property value is less than a minimum acceptable property value, then the scored property value is one (1).

19. The method of clause 2, wherein if at least one input parameter obtained from the user is minimize the property value, and if the actual property value is not greater than a maximum acceptable property value, and if the actual property value is not less than a minimum acceptable property value, then the scored property value may be calculated using the desirability function:

$$Score = \left[ \frac{MAX - APV}{MAX - MIN} \right]^X$$

wherein APV = actual property value, MAX = user-specified maximum acceptable property value, MIN = user-specified minimum acceptable property value, and x = the weight value.

20. The method of clause 2, wherein if at least one input parameter obtained from the user is hit a target point value for the property value, and if:

$$APV \geq DPV \text{ and } APV \leq MAX$$

then the scored property value may be calculated using the desirability function:

$$Score = \left[ \frac{MAX - APV}{MAX - DPV} \right]^X$$

wherein APV = actual property value, MAX = user-specified maximum acceptable property value, MIN = user-specified minimum acceptable property value, DPV = user-specified desired property value, and x = the weight value.

21. The method of clause 2, wherein if at least one input parameter obtained from the user is hit a target point value for the property value, and if the actual property value is greater than a maximum acceptable property value, then the scored property value is zero (0).

22. The method of clause 2, wherein if at least one input parameter obtained from the user is hit a target point value for the property value, and if:

$$APV \leq DPV \text{ and } APV \geq MIN$$

then the scored property value may be calculated using the desirability function:

$$Score = \left[ \frac{APV - MIN}{DPV - MIN} \right]^X$$

wherein APV = actual property value, MAX = user-specified maximum acceptable property value, MIN = user-specified minimum acceptable property value, DPV = user-specified desired property value, and x = the weight value.

23. The method of clause 2, wherein if at least one input parameter obtained from the user is hit a target point value for the property value, and if the actual property value is less than a minimum acceptable property value, then the scored property value is zero (0).

24. The method of clause 2, wherein if at least one input parameter obtained from the user is to keep the property value within a given range of acceptable property values, and if MIN ≤ APV ≤ MAX, wherein APV = actual property value, MAX = user-specified maximum acceptable property value, and MIN = user-specified minimum acceptable property value, then the scored property value is one (1).

25. The method of clause 2, wherein if at least one input parameter obtained from the user is to keep the property value within a given range of acceptable property values, and if APV > MAX or APV < MIN, wherein APV = actual property value, MAX = user-specified maximum acceptable property value, and MIN = user-specified minimum acceptable property value, then the scored property value is zero (0).

26. The method of clause 13, wherein if a high priority exists for a property, a priority value of 5 is assigned to that property, if a medium priority exists for a property, a priority value of 3 is assigned to that property, and if a low priority exists for a property, a priority value of 1 is assigned to that property.

27. The method of clause 2, wherein the overall match score is calculated using the formula:

$$OverallMatchScore=\left[\prod_{i=1}^{n}\left(Score_i\right)^{priority_i}\right]^{1/\sum_{i=1}^{n}priority}$$

28. The method of clause 1, wherein each preliminary matching experimental run comprises an engineering thermoplastic.

29. The method of clause 28, wherein the engineering thermoplastic comprises at least one of a polyester, polyethylene terephthalate (PET), polybutylene terephthalate (PBT), polyethylene naphthalate (PEN), liquid crystal polyester (LCP), a polyolefin, polyethylene (PE), polypropylene (PP), polybutylene, a styrene-type resin, polyoxymethylene (POM), polyamide (PA), polycarbonate (PC), polymethylene methacrylate (PMMA), polyvinyl chloride (PVC), polyphenylene sulfide (PPS), polyphenylene ether (PPE), polyimide (PI), polyamide imide (PAI), polyetherimide (PEI), polysulfone (PSU), polyether sulphone (PES), polyketone (PK), polyether ketone (PEK), polyether ether ketone (PEEK), polyalylate (PAR), polyethernitrile (PEN), a phenol resin (novolac type), a phenoxy resin, a fluorocarbon resin, a thermoplastic elastomer of a polystyrene type, a thermoplastic elastomer of a polyolefin type, a thermoplastic elastomer of a polyurethane type, a thermoplastic elastomer of a polyester type, a thermoplastic elastomer of a polyamide type, a thermoplastic elastomer of a polybutadiene type, a thermoplastic elastomer of a polyisoprene type, and a thermoplastic elastomer of a fluorine type.

30. The method of clause 28, wherein the engineering thermoplastic comprises at least one of a styrene-type resin, a polycarbonate resin, a polyphenylene ether resin, a polyamide resin, a polyester resin, a polyphenylene sulfide resin, a liquid-crystalline resin and a phenol-type resin.

31. A system for designing a material that most closely matches a desired set of properties, the system comprising:

a means for obtaining at least one input parameter from a user;
a means for retrieving actual property values for at least one preliminary matching existing experimental run from a global data repository;
a material selection algorithm operable for determining how well each preliminary matching existing experimental run matches a desired set of property values; and

a means for outputting the results to the user.

32. The system of clause 31, wherein the material selection algorithm is further operable for: scoring each property value of each preliminary matching existing experimental run to create a scored property value; and calculating an overall match score for each preliminary matching existing experimental run.

33. The system of clause 32, wherein the material selection algorithm is further operable for:

sorting the preliminary matching existing experimental runs by their respective overall match scores prior to outputting the results to the user.

34. The system of clause 33, wherein the preliminary matching existing experimental runs are sorted in descending order based on their respective overall match scores.

35. The system of clause 31, further comprising:

a material prediction algorithm operable for predicting at least one new material that may more closely match the desired set of properties than any existing experimental run.

36. The system of clause 32, wherein the material selection algorithm is further operable for:

scoring each property value of each new material to create a scored property value; and calculating an overall match score for each new material.

37. The system of clause 36, wherein the material selection algorithm is further operable for:

sorting the preliminary matching existing experimental runs and the new materials by their respective overall match scores prior to outputting the combined results thereof to the user.

38. The method of clause 35, wherein the predicting step comprises applying a transfer function to predict the new material that may more closely match the desired set of properties than any existing experimental run.

39. The system of clause 31, wherein the at least one input parameter comprises at least one of: a specific raw material to search for, a design space to retain for advanced searching, a design space to retain for scoring, a property to be searched, units for each property to be searched, acceptable property values for each property to be searched, a goal for each property to be searched, and a priority value for each property to be searched.

40. The system of clause 39, wherein the goal for each property to be searched comprises at least one of: maximize the property value, minimize the property value, hit a target point value for the property value, and keep the property value within a given range of acceptable property values.

41. The system of clause 39, wherein the priority value for each property to be searched comprises at least one of: high, medium and low.

42. The system of clause 32, wherein if at least one input parameter obtained from the user is maximize the property value, and if the actual property value is less than a minimum acceptable property value, then the scored property value is zero (0).

43. The system of clause 32, wherein if at least one input parameter obtained from the user is maximize the property value, and if the actual property value is greater than a maximum acceptable property value, then the scored property value is one (1).

44. The system of clause 32, wherein if at least one input parameter obtained from the user is maximize the property value, and if the actual property value is not less than a minimum acceptable property value, and if the actual property value is not greater than a maximum acceptable property value, then the scored property value may be calculated using the desirability function:

$$\text{Score} = \left[\frac{\text{APV} - \text{MIN}}{\text{MAX} - \text{MIN}}\right]^{\text{X}}$$

wherein APV = actual property value, MAX = user-specified maximum acceptable property value, MIN = user-specified minimum acceptable property value, and x = the weight value.

45. The system of clause 32, wherein if at least one input parameter obtained from the user is minimize the property value, and if the actual property value is greater than a maximum acceptable property value, then the scored property value is zero (0).

46. The system of clause 32, wherein if at least one input parameter obtained from the user is minimize the property value, and if the actual property value is less than a minimum acceptable property value, then the scored property value is one (1).

47. The system of clause 32, wherein if at least one input parameter obtained from the user is minimize the property value, and if the actual property value is not greater than a maximum acceptable property value, and if the actual property value is not less than a minimum acceptable property value, then the scored property value may be calculated using the desirability function:

$$\text{Score} = \left[\frac{\text{MAX} - \text{APV}}{\text{MAX} - \text{MIN}}\right]^{\text{X}}$$

wherein APV = actual property value, MAX = user-specified maximum acceptable property value, MIN = user-specified minimum acceptable property value, and x = the weight value.

48. The system of clause 32, wherein if at least one input parameter obtained from the user is hit a target point value for the property value, and if:

$$\text{APV} \geq \text{DPV and APV} \leq \text{MAX}$$

then the scored property value may be calculated using the desirability function:

$$\text{Score} = \left[\frac{\text{MAX} - \text{APV}}{\text{MAX} - \text{DPV}}\right]^{\text{X}}$$

wherein APV = actual property value, MAX = user-specified maximum acceptable property value, MIN = user-specified minimum acceptable property value, DPV = user-specified desired property value, and x = the weight value.

49. The system of clause 32, wherein if at least one input parameter obtained from the user is hit a target point value for the property value, and if the actual property value is greater than a maximum acceptable property value, then the scored property value is zero (0).

50. The system of clause 32, wherein if at least one input parameter obtained from the user is hit a target point value for the property value, and if:

$$\text{APV} \leq \text{DPV and APV} \geq \text{MIN}$$

then the scored property value may be calculated using the desirability function:

$$Score = \left[\frac{APV - MIN}{DPV - MIN}\right]^{X}$$

wherein APV = actual property value, MAX = user-specified maximum acceptable property value, MIN = user-specified minimum acceptable property value, DPV = user-specified desired property value, and x = the weight value.

51. The system of clause 32, wherein if at least one input parameter obtained from the user is hit a target point value for the property value, and if the actual property value is less than a minimum acceptable property value, then the scored property value is zero (0).

52. The system of clause 32, wherein if at least one input parameter obtained from the user is to keep the property value within a given range of acceptable property values, and if MIN ≤ APV ≤ MAX, wherein APV = actual property value, MAX = maximum acceptable property value, and MIN = minimum acceptable property value, then the scored property value is one (1).

53. The system of clause 32, wherein if at least one input parameter obtained from the user is to keep the property value within a given range of acceptable property values, and if APV > MAX or APV < MIN, wherein APV = actual property value, MAX = maximum acceptable property value, and MIN = minimum acceptable property value, then the scored property value is zero (0).

54. The system of clause 41, wherein if a high priority exists for a property, a priority value of 5 is assigned to that property, if a medium priority exists for a property, a priority value of 3 is assigned to that property, and if a low priority exists for a property, a priority value of 1 is assigned to that property.

55. The system of clause 32, wherein the overall match score is calculated using the formula:

$$OverallMatchScore = \left[\prod_{i=1}^{n}\left(Score_i\right)^{priority_i}\right]^{1/\sum_{i=1}^{n}priority}$$

56. The system of clause 31, wherein each preliminary matching experimental run comprises an engineering thermoplastic.

57. The system of clause 56, wherein the engineering thermoplastic comprises at least one of a polyester, polyethylene terephthalate (PET), polybutylene terephthalate (PBT), polyethylene naphthalate (PEN), liquid crystal polyester (LCP), a polyolefin, polyethylene (PE), polypropylene (PP), polybutylene, a styrene-type resin, polyoxymethylene (POM), polyamide (PA), polycarbonate (PC), polymethylene methacrylate (PMMA), polyvinyl chloride (PVC), polyphenylene sulfide (PPS), polyphenylene ether (PPE), polyimide (PI), polyamide imide (PAI), polyetherimide (PEI), polysulfone (PSU), polyether sulphone (PES), polyketone (PK), polyether ketone (PEK), polyether ether ketone (PEEK), polyalylate (PAR), polyethernitrile (PEN), a phenol resin (novolac type), a phenoxy resin, a fluorocarbon resin, a thermoplastic elastomer of a polystyrene type, a thermoplastic elastomer of a polyolefin type, a thermoplastic elastomer of a polyurethane type, a thermoplastic elastomer of a polyester type, a thermoplastic elastomer of a polyamide type, a thermoplastic elastomer of a polybutadiene type, a thermoplastic elastomer of a polyisoprene type, and a thermoplastic elastomer of a fluorine type.

58. The system of clause 56, wherein the engineering thermoplastic comprises at least one of a styrene-type resin, a polycarbonate resin, a polyphenylene ether resin, a polyamide resin, a polyester resin, a polyphenylene sulfide resin, a liquid-crystalline resin and a phenol-type resin.

59. A product formulated by the process comprising:

obtaining at least one input parameter from a user;
retrieving actual property values for at least one preliminary matching existing experimental run from a global data repository;
determining how well each preliminary matching existing experimental run matches a desired set of property values;
outputting the results to the user; and
using the results to design a product that best meets the at least one input parameter from the user.

60. The product of clause 59, wherein the determining step comprises:

scoring each property value of each preliminary matching existing experimental run to create a scored property value; and
calculating an overall match score for each preliminary matching existing experimental run.

61. The product of clause 60, wherein calculating an overall match score comprises:

weighting each scored property value by taking a weight value for each property into account to create a weighted scored property value;
multiplying each weighted scored property value together; and
raising the multiplied quantity to 1/(sum of all the priorities).

62. The product of clause 60, further comprising:

sorting the preliminary matching existing experimental runs by their respective overall match scores prior to outputting the results to the user.

63. The product of clause 62, wherein the preliminary matching existing experimental runs are sorted in descending order based on their respective overall match scores.

64. The product of clause 59, further comprising the step of:

predicting at least one new material that may more closely match the desired set of properties than any existing experimental run.

65. The product of clause 64, further comprising the steps of:

scoring each property value of each new material to create a scored property value; and calculating an overall match score for each new material.

66. The product of clause 65, wherein calculating an overall match score comprises:

weighting each scored property value by taking a weight value for each property into account to create a weighted scored property value;
multiplying each weighted scored property value together; and
raising the multiplied quantity to 1/(sum of all the priorities).

67. The product of clause 65, further comprising the step of:

sorting the preliminary matching existing experimental runs and the new materials by their respective overall match scores prior to outputting the combined results thereof to the user.

68. The product of clause 64, wherein the predicting step comprises applying a transfer function to predict the new material that may more closely match the desired set of properties than any existing experimental run.

69. The product of clause 59, wherein the product comprises at least one of an experimental grade engineering thermoplastic material, a developmental grade engineering thermoplastic material, and a commercial grade engineering thermoplastic material.

70. The product of clause 59, wherein the product comprises at least one of a polyester, polyethylene terephthalate (PET), polybutylene terephthalate (PBT), polyethylene naphthalate (PEN), liquid crystal polyester (LCP), a polyolefin, polyethylene (PE), polypropylene (PP), polybutylene, a styrene-type resin, polyoxymethylene (POM), polyamide (PA), polycarbonate (PC), polymethylene methacrylate (PMMA), polyvinyl chloride (PVC), polyphenylene sulfide (PPS), polyphenylene ether (PPE), polyimide (PI), polyamide imide (PAI), polyetherimide (PEI), polysulfone (PSU), polyether sulphone (PES), polyketone (PK), polyether ketone (PEK), polyether ether ketone (PEEK), polyalylate (PAR), polyethernitrile (PEN), a phenoxy resin, a fluorocarbon resin, a thermoplastic elastomer of a polystyrene type, a thermoplastic elastomer of a polyolefin type, a thermoplastic elastomer of a polyurethane type, a thermoplastic elastomer of a polyester type, a thermoplastic elastomer of a polyamide type, a thermoplastic elastomer of a polybutadiene type, a thermoplastic elastomer of a polyisoprene type, a thermoplastic elastomer of a fluorine type, a styrene-type resin, a polyphenylene ether resin, a polyamide resin, a polyphenylene sulfide resin, a liquid-crystalline resin and a phenol-type resin.

**Claims**

1. A method for designing a material that most closely matches a desired set of properties, the method comprising:

   obtaining at least one input parameter from a user;
   retrieving actual property values for at least one preliminary matching existing experimental run from a global data repository;
   determining how well each preliminary matching existing experimental run matches a desired set of property values; and
   outputting the results to the user.

2. The method of clause 1, wherein the determining step comprises:

   scoring each property value of each preliminary matching existing experimental run to create a scored property value; and
   calculating an overall match score for each preliminary matching existing experimental run.

3. The method of clause 2, wherein calculating an overall match score comprises:

   weighting each scored property value by taking a weight value for each property into account to create a weighted scored property value;
   multiplying each weighted scored property value together; and
   raising the multiplied quantity to 1/(sum of all the priorities).

4. The method of clause 2, further comprising:

   sorting the preliminary matching existing experimental runs by their respective overall match scores prior to outputting the results to the user.

5. The method of clause 4, wherein the preliminary matching existing experimental runs are sorted in descending order based on their respective overall match scores.

6. The method of clause 1, further comprising the step of:

   predicting at least one new material that may more closely match the desired set of properties than any existing experimental run.

7. The method of clause 6, further comprising the steps of:

   scoring each property value of each new material to create a scored property value; and calculating an overall match score for each new material.

**8.** The method of clause 7, wherein calculating an overall match score comprises:

weighting each scored property value by taking a weight value for each property into account to create a weighted scored property value;
multiplying each weighted scored property value together; and
raising the multiplied quantity to 1/(sum of all the priorities).

**9.** The method of clause 7, further comprising the step of:

sorting the preliminary matching existing experimental runs and the new materials by their respective overall match scores prior to outputting the combined results thereof to the user.

**10.** A system for designing a material that most closely matches a desired set of properties, the system comprising:

a means for obtaining at least one input parameter from a user;
a means for retrieving actual property values for at least one preliminary matching existing experimental run from a global data repository;
a material selection algorithm operable for determining how well each preliminary matching existing experimental run matches a desired set of property values; and
a means for outputting the results to the user.

**FIGURE 1**

**FIGURE 2**

**FIGURE 3**

**FIGURE 4**

**FIGURE 5**

**FIGURE 6**